(19) Europäisches Patentamt
European Patent Office
Office européen des brevets

(11) Publication number: **0 367 606 A1**

# (12) EUROPEAN PATENT APPLICATION

(21) Application number: **89311359.7**

(51) Int. Cl.5: **C07D 499/86**

(22) Date of filing: **02.11.89**

(30) Priority: **04.11.88 IT 2252488**

(43) Date of publication of application: **09.05.90 Bulletin 90/19**

(84) Designated Contracting States: **AT BE CH DE ES FR GB LI NL SE**

(71) Applicant: **GLAXO S.p.A.**
**Via Fleming, 2**
**I-37100 Verona(IT)**

(72) Inventor: **Gaviraghi, Giovanni**
**Via G.B. Pergolesi n.13**
**I-37131 Verona(IT)**
Inventor: **Tamburini, Bruno Via Don Fantoni n.2**
**I-37020 S. Floriano di S. Pietro in Cariano Verona(IT)**
Inventor: **Pellicciari, Roberto**
**Via Ulisse Rocchi n.60**
**I-06100 Perugia(IT)**

(74) Representative: **James, Stephen Richard**
**Glaxo Holdings plc Patents/Trade Marks Division Graham Street**
**London N1 8JZ(GB)**

## (54) Process for the preparation of 6-oxopenicillanates.

(57) A process is provided for the preparation of compounds of formula (I)

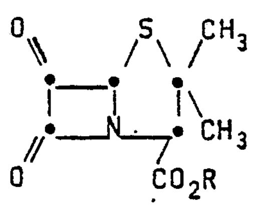

(I)

which comprises ozonization of a solution of a diazo compound of formula (II)

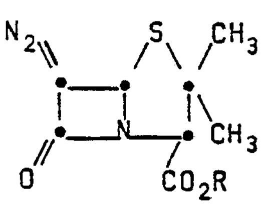

(II)

at a temperature below 0° C in an organic soylent.

EP 0 367 606 A1

## PROCESS FOR THE PREPARATION OF 6-OXOPENICILLANATES

This invention relates to a process for the preparation of 6-oxopenicillanates.

6-oxopenicillanates are known intermediates for the preparation of therapeutically useful penicillin derivatives.

Several methods are described in the literature for the preparation of 6-oxopenicillanates, a number of which involve the oxidation of the corresponding 6-hydroxypenicillanates, for example, using dimethylsulphoxide with a carbodiimide or dimethylsulphoxide with acetic anhydride. The 6-hydroxypenicillanic derivatives may be prepared from the 6-diazo derivatives by hydrolysis, for example using perchloric acid in aqueous acetone.

We have found a novel and improved route for the preparation of 6-oxopenicillanates which not only utilizes readily available starting materials and reactants but the reaction conditions are such that the required product may be readily isolated from the reaction medium, or if desired, used in subsequent reactions without purification and/or isolation from the reaction medium.

According to one aspect of the present invention, there is provided a process for the preparation of the compounds of formula

(I)

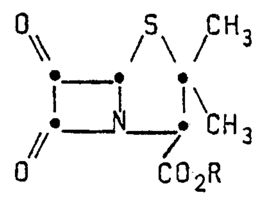

(I)

(wherein R represents a carboxyl protecting group) which comprises ozonization of a diazo compound of formula (II)

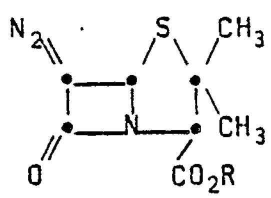

(II)

(wherein R represents a carboxyl protecting group). Examples of suitable carboxyl protecting groups include those described in 'Protective Groups in Organic Chemistry' Ed. J.W. McOmie (Plenum Press, 1973) or 'Protective Groups in Organic Synthesis' by Theodora W. Greene (John Wiley and Sons, 1981). Thus, for example, the group $-CO_2R$ may represent an ester group in which R may represent an optionally substituted alkyl group, such as $C_{1-6}$alkyl, arylmethyl, aroylmethyl, alkoxymethyl, acyloxymethyl or a 2,2,2-trichloroethyl group.

Thus R may represent a $C_{1-6}$alkyl (e.g. methyl or t-butyl), arylmethyl (e.g. phenylmethyl, diphenylmethyl, cumyl or p-nitrophenylmethyl), aroylmethyl (e.g. p-bromophenacyl), alkoxymethyl (e.g. methoxymethyl), acyloxymethyl such as alkanoyloxymethyl (e.g. pivaloyloxymethyl), or a 2,2,2-trichloroethyl group.

Preferably R represents a $C_{1-6}$alkyl group (e.g. methyl or t-butyl), an arylmethyl group (e.g. phenylmethyl, diphenylmethyl or p-nitrophenylmethyl), an acyloxymethyl such as an alkanoyloxymethyl group (e.g. pivaloyloxymethyl) or a 2,2,2-trichloroethyl group.

The reaction may be effected by passing a stream of ozone, conveniently in the form of ozone-enriched oxygen, into a solution of a compound of formula (II) in a suitable solvent such as a hydrocarbon (e.g. n-pentane or n-hexane), a halogenated hydrocarbon (e.g. dichloromethane or carbon tetrachloride), an ester (e.g. ethyl acetate) or an alcohol (e.g. methanol) at a temperature in the range of -80 to 0 °C, preferably -30 to -10 °C.

The above described process provides compounds of formula (I) in good yield and high purity. The

compounds of formula (I) thus obtained may be readily isolated from the reaction medium or, if desired, used in subsequent reactions without isolation from the reaction medium.

Compounds of formula (II) may be prepared by the diazotization of a 6-aminopenicillanate of formula (III)

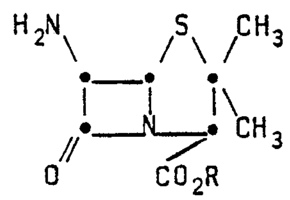

(III)

(where R represents a carboxyl protecting group as defined above) or suitable salt thereof according to conventional methods described in the art.

For example, the reaction may be affected by treating a compound of formula (III) or a suitable salt thereof (e.g. tosylate) with nitrous acid or isoamyl nitrite in the presence of acid conveniently in a solvent such as a halogenated hydrocarbon (e.g. dichloromethane) at a temperature in the range of -10 to +10 °C.

The 6-aminopenicillanates of formula (III) are either known compounds or may be prepared from readily available 6-aminopenicillanic acid by esterification according to methods well-known in the art.

The diazo compounds of formula (II) may be used in the ozonization reaction without the need for prior purification and/or isolation from the reaction mixture.

Thus, according to one embodiment of the present invention these is provided a process for the preparation of the compounds of formula (I) which comprises ozonization of a diazo compound of formula (II) which has been prepared in situ from the 6-aminopenicillanate of formula (III) or a suitable salt thereof using the reaction conditions described above.

If desired the compound of formula (I) thus obtained may be used in subsequent reactions without further purification and/or isolation from the reaction medium.

The invention is illustrated but not limited by the following examples in which temperatures are in °C.

## Example 1

(2S, 5R)-6-oxo-3,3-dimethyl-7-oxo-4-thia-1-azobicyclo-[3,2,0]heptane-2 (pivaloyloxymethylcarboxylate)

A solution of pivaloyloxymethyl 6-diazopenicillanate (4.71 mmoles) in methylene chloride (100 ml) was ozonized at -10 to -15° with an ozone-oxygen stream. Evaporation of the reaction mixture to dryness under reduced pressure gave the title compound (1.44 g), $\nu_{max}$ ($CDCl_3$) 1834 (C=0, ketone), 1792 (C=0, beta lactam), 1763 (C=0, esters) $cm^{-1}$; $\delta$ ($CDCl_3$) 5.84 (3H, m, $-O-CH_2-O-$ and -CH-S-), 4.83 (1H, s, $-N-CH-CO_2-$), 1.56 (6H, s, $-CH_3$), 1.23 (9H, s, $-C(CH_3)_3$).

## Example 2

(2S, 5R)-6-oxo-3,3-dimethyl-7-oxo-4-thia-1-azabicyclo-[3,2,0]-heptane-2 (diphenylmethylcarboxylate)

A solution of diphenylmethyl 6-diazopenicillanate (4.93 mmoles) in methylene chloride (100 ml) was ozonized at -10° to -15° with an ozone-oxygen stream. Evaporation of the reaction mixture to dryness under reduced pressure gave the title compound (1.68 g), $\nu_{max}$($CDCl_3$) 1830 (C=0, ketone), 1792 (C=0, beta lactam), 1747 (C=0, ester)$cm^{-1}$; $\delta$ ($CDCl_3$) 7.34 (10H, s, aromatics), 6.98 (1H, bs, $-(Ph_2)CH-$), 5.80 (1H, s, -CH-S-), 4.87 (1H, s, $-N-CH-CO_2-$), 1.54 (3H, s, $CH_3$), 1.34 (3H, s, $CH_3$)

## Claims

1. A process for the preparation of the compounds of formula (I)

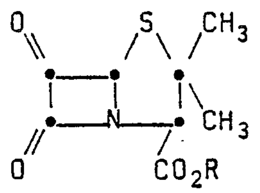

(I)

wherein R represents a carboxyl protecting group which comprises ozonization of a diazo compound of formula (II)

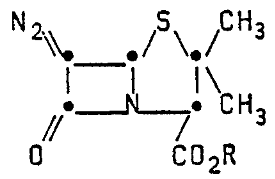

wherein R is as defined above.

2. A process as claimed in claim 1 for the preparation of compounds of formula (I) wherein R represents an optionally substituted alkyl group.

3. A process as claimed in claim 2 for the preparation of compounds of formula (I) wherein R represents a group selected from $C_{1-6}$alkyl, arylmethyl, aroylmethyl, alkoxymethyl, acyloxymethyl or 2,2,2-trichloroethyl.

4. A process as claimed in claim 3 for the preparation of compounds of formula (I) wherein R represents a group selected from $C_{1-6}$alkyl, arylmethyl, alkanoyloxymethyl or 2,2,2-trichloroethyl.

5. A process as claimed in claim 4 for the preparation of compounds of formula (I) wherein R represents a group selected from methyl, t-butyl, phenylmethyl, diphenylmethyl, p-nitrophenylmethyl, pivaloyloxymethyl or 2,2,2-trichloroethyl.

6. A process as claimed in any of claims 1 to 5 wherein ozonization is effected using ozone-enriched oxygen.

7. A process as claimed in any of claims 1 to 6 wherein ozonization is carried out in a solvent selected from a hydrocarbon, halogenated hydrocarbon, ester or alcohol.

8. A process as claimed in any of claims 1 to 7 wherein ozonization is carried out at a temperature in the range of -80 to 0 °C.

9. A process for the preparation of a compound of formula (I) as claimed in any of claims 1 to 8 wherein the diazo compound of formula (II) is prepared by diazotization of the corresponding 6-aminopenicillanate of formula (III)

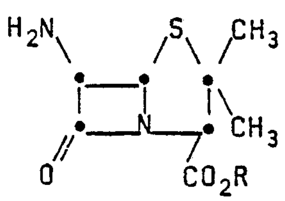

(III)

wherein R is as defined in any of claims 1 to 5, or a suitable salt thereof.

10. A compound of formula (I) when prepared by a process as claimed in any of claims 1 to 9.

European Patent Office

# EUROPEAN SEARCH REPORT

Application Number

EP 89 31 1359

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | US-A-4 053 468 (K.G. HOLDEN)<br>* Claims * | 1-5,9,<br>10 | C 07 D 499/86 |
| Y | JOURNAL OF ORGANIC CHEMISTRY, vol. 42, no. 25, 9th December 1977, pages 4045-4048; J.C. SHEEHAN et al.: "Derivatives of 6beta-Methylpenicillanic acid"<br>* Whole article * | 1-5,9,<br>10 | |
| Y | US-A-4 143 046 (J.C. SHEEHAN et al.)<br>* Column 2, paragraph 2; claims * | 1-5,9,<br>10 | |
| A | EP-A-0 043 546 (FUJISAWA)<br>* Claims * | 1-5 | |
| A | CHEMICAL ABSTRACTS, vol. 105, no. 13, 29th September 1986, page 650, abstract no. 114785b, Columbus, Ohio, US; D. HAEBICH et al.: "Inhibitors of beta-lactamases. 2. Synthesis of 6-Sulfonylmethlene-, 6-sulfinylmethylene- and spiropyrazo=line-penicillianic acids", & HETEROCYCLES 1986, 24(2), 289-96<br>* Abstract * | 1-5 | TECHNICAL FIELDS SEARCHED (Int. Cl.5)<br>C 07 D 499/00 |

The present search report has been drawn up for all claims

| Place of search | Date of completion of the search | Examiner |
|---|---|---|
| THE HAGUE | 30-01-1990 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)